# EUROPEAN PATENT APPLICATION

(11) **EP 1 634 613 A1**
(43) Date of publication of application: **15.03.2006**
(21) Application number: 05019516.3
(22) Date of filing: 08.09.2005
(51) Int. Cl.: A61M 5/142, A61M 5/00

(54) **Apparatus for carrying out haematic infusions**

(30) Priority: 10.09.2004 IT BO20040554
(71) Applicant: S.I.A.S. S.p.A., 40057 Cadriano di Granarolo Emilia BO (IT)
(72) Inventor: Bonetti, Fabio, 40122 Bologna (IT)
(74) Representative: Dall'Olio, Giancarlo

(57) **Abstract**

The apparatus for carrying out haematic infusions comprises: a casing (101); a support (102) for one or more containers (103,104) of infusion product, associated to the casing (101); a disposable pumping group (1), supported by the casing (101) and removable from and mountable on the same casing (101).

The pumping group (1), in turn, comprises a reciprocating volumetric pump (2,2a), provided with one pair of cylinders (3,3a) each one receiving a piston (4,4a). A collecting device (10) for collecting the infusion product from containers (103,104) is fit to penetrate the same containers (103,104) and to collect the infusion product therefrom. A connecting pipe (20) extends from the collecting device (10) and an input joint (6,6a) of the volumetric pump (2,2a). A unidirectional input valve (7,7a) is interposed between each input joint (6,6a) and each cylinder (3,3a), in order to allow the infusion product to flow exclusively toward the same cylinder (3,3a). An output pipe (30) is connected to an output joint (8,8a) of the volumetric pump (2,2a), for conveying infusion products from the cylinders (3,3a) to means for injecting the product into a vein. A unidirectional output valve (9,9a) is interposed between each one of said cylinders (3,3a) and each output joint (8,8a), in order to allow the infusion product to flow exclusively away from the same cylinders (3,3a).

The infusion apparatus (100) moreover comprises actuating means (40), connected to the aforesaid pistons (4,4a) for reciprocally operating them according to predefined operating modes.

## Description

The present invention fits into the technical sector concerning electromedical equipment.

More particularly, the invention relates to an apparatus fit to carrying out intravenous infusions of liquid products as, e.g., physiological substances or contrast means, mostly suitable for diagnostic purposes.

It is known that the intravenous infusion of liquid products, like by example those containing substances to be used as a radioopaque contrast means for radioscopical examinations on soft tissues, is mainly carried out with two different kinds of infusion apparatus.

A first kind of such apparatus comprises, in the most schematic structure thereof, a peristaltic pump, fit to create a positive pressure in a hose made of resilient material. Such hose is filled with the liquid to be injected, which is contained in a bottle. The hose is connected to the bottle by means of a suitable collecting joint provided at one hose end. At the opposite end, the hose comprises a needle, or any other similar device fit to puncture the blood vessel wherein the liquid product has to be injected.

Such peristaltic pump, whose operating procedure is perfectly known to every skilled operator, allows to create a pressure inside the hose which is greater than the blood pressure, and which also compensates the pressure drops throughout the hose itself. It also pushes the liquid in the hose toward the correct injection way.

The above described apparatus is advantageous in that the liquid is compressed without any contact between the liquid itself and the pump. Therefore there is no need for any pump component to be sterilized after every infusion session.

On the other hand, an evident drawback of a such kind of infusion apparatus is that a peristaltic pump cannot compress a liquid to pressures exceeding 20 atmospheres. Yet several commonly used infusion products containing a contrast means have a somewhat high viscosity, which results in remarkable pressure drops along the product path to vein. The highest pressures achievable with peristaltic pumps are not therefore high enough to guarantee a correct infusion of the aforesaid products, which need at least 26 ― 27 athmospheres (approx. 400 psi) to be correctly injected.

A further known drawback of the aforementioned infusion apparatus resides in the strong pressure module oscillations occurring to the infusion liquid inside the hoses, due to the substantially discontinuous operating mode of the peristaltic pumps. These apparatus give, as a matter of fact, a highly pulsatile flow, mostly for high infusion speeds and pressures.

Another kind of known infusion apparatus comprises a volumetric pump including a syringe-like pump. A pump outlet is connected to a hose end carrying, at its opposite end, the needle to be inserted into a vein. Inside the cylindrical pump body a pneumatic-actuated piston is provided, for compressing a given amount of infusion liquid contained in the same cylindrical body.

The so structured volumetric pump is able to compress the infusion product up to pressures exceeding 80 atmospheres (approx. 1500 psi), thus allowing infusion products with very high viscosity degree to be used for diagnostic purposes. Moreover, this pump has a continuous operating mode, and this gives a substantially constant pressure module during infusion.

A major drawback of the aforesaid kind of volumetric pump is that the maximum amount of infusion product which can be injected in vein with a single session is limited by the volume of the pump body. This latter has to be filled every time with the infusion product, and the infusion operation has to be suspended when the cylindrical pump body empties. In order to restart the infusion process, the pump must be disassembled and filled again with the product to be injected.

A further drawback arises from the fact that, since the product to be injected is in contact with the pump body, this latter must be sterilized each time it is used, or at least it must be accurately cleaned after each session. This produces working time losses, and makes the infusion apparatus not readily reusable, resulting in remarkable additional costs in a long-period operation.

The main object of the present invention is to provide an apparatus for carrying out haematic infusions which is able to operate at injection pressures considerably higher than those achievable with the peristaltic pumps, yet also operating with a continuous flow and with substantially unlimited amounts of the infusion product.

A further object of the invention is to provide an apparatus which is able to allow the same apparatus to be readily reusable for subsequent infusion sessions.

Another object of the invention is to provide an apparatus which allows to inject, either alternately or together, more than one infusion product during the same infusion session.

Yet another object of the invention is to provide an apparatus which is structurally simple, reliable and featuring low operating costs.

All the aforesaid objects are fully achieved, according to the contents of the appended Claims.

All the characteristic features of the present invention, as they will result from the Claims, are pointed out in the following detailed description, with reference to the enclosed drawing tables, wherein:
- figure 1 shows a front, partially broken view, of the infusion apparatus made according the teachings of the present invention, with some apparatus components shown sectionally;
- figure 2 shows a side sectional view of the apparatus of figure 1;
- figure 3 shows a front view of the pumping group belonging to the apparatus of figures 1 and 2.

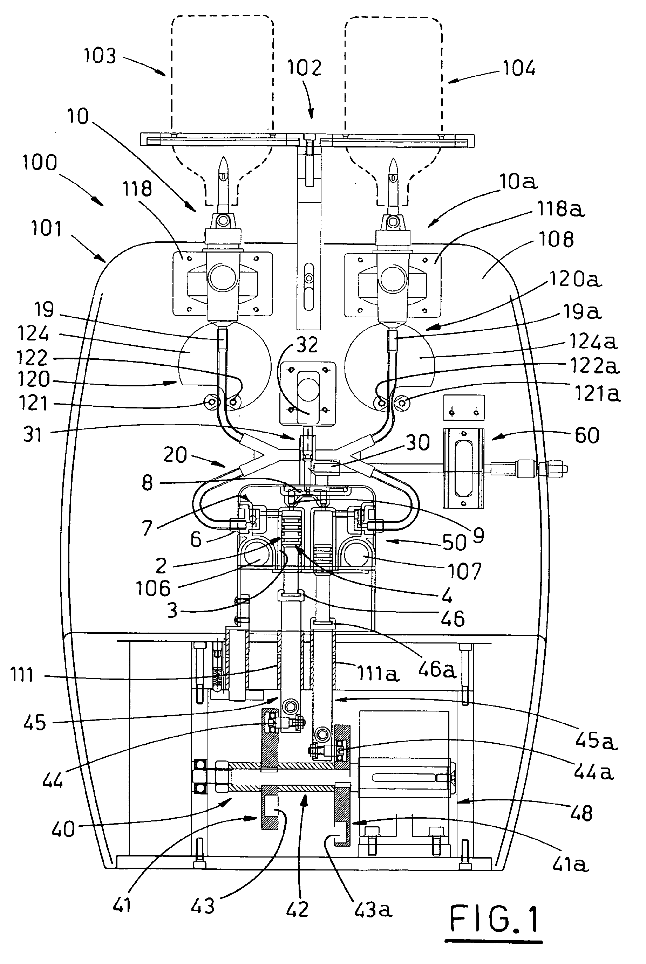

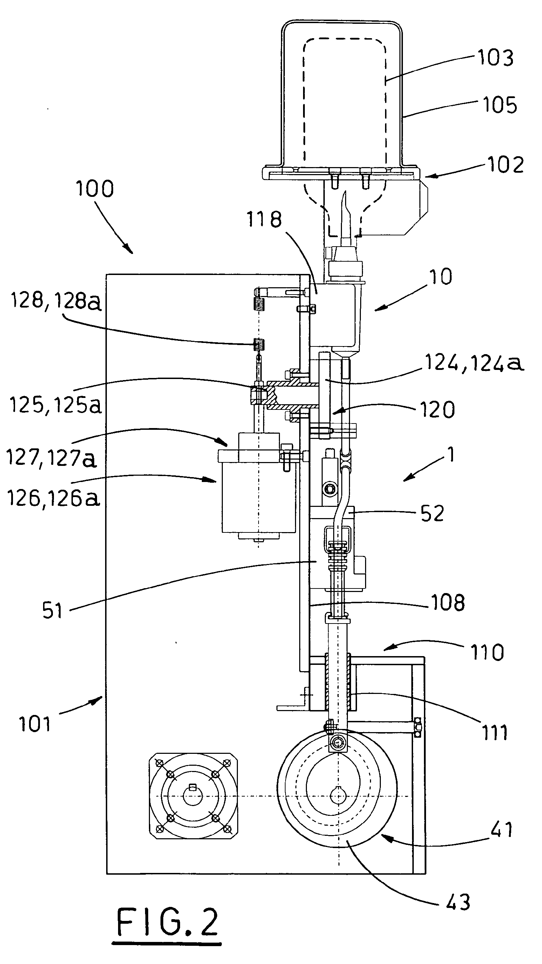

Referring now to figures 1 and 2, numeral 100 indicates, as a whole, an apparatus for carrying out infusions of liquid products into a vein, said products comprising, by example, a radio-opaque contrast means used in radioscopical examinations on soft tissues. This apparatus 100 comprises, in a preferred but non exclusive embodiment, a closed casing 101, made of metal or any other material having suitable mechanical and aesthetical characteristic features, and also provided with known electromagnetic protection devices.

The aforesaid casing 101 comprises a substantially vertical front wall 108, fit to support on its outer face some components specifically belonging to said apparatus 100, as it will better specified further on.

A support 102 for a pair of containers 103,104 for an infusion liquid is secured to the upper outer part of the front wall 108. Containers 103,104 are usually glass bottles, which are arranged upside down on their support 102. In figure 2 is moreover shown a covering ad heating body 105 for the same containers 103,104, which can be omitted if not necessary.

The apparatus 100 moreover comprises a pumping group 1 (see also figure 1), fit to take contrast liquid from containers 103,104, to compress it and to send it toward known means for injecting said contrast liquid into a vein, not shown, as they are the so called "butterfly" type needles, according to techniques which will be described in detail later on. This pumping group 1, more particularly, is being disclosed in the copending patent application N. BO2004A000555 filed on this day by the same Applicant.

The pumping group 1, in the preferred embodiment cited above, is movably mounted on the outer front wall 108 of the casing 101and, as it will clearly appear, is fit to be easily mounted and unmounted in order to be substituted with a new and perfectly sterile one.

The pumping group 1 comprises a pair of reciprocating volumetric pumps 2,2a, whose bodies, which are arranged parallel one each other, are made in a single block 50 of a moulded plastic material, formed by two half-shells 51,52, which are fixed one each other with known gluing or soldering techniques. This single block 50 moreover comprises a pair of through holes 53,54, fit to receive corresponding pins 106,107 extending from the aforementioned front wall 108, in order to held in its operating position the same block 50.

Each volumetric pump 2,2a respectively comprises one cylinder 3,3a which receives a corresponding piston 4,4a fit to reciprocally slide inside the piston in a liquid-tight way. The liquid-tight condition is achieved by means of suitable O-ring gaskets. When in their operating position, the volumetric pumps 2,2a are arranged so as to stay substantially vertical.

The pumping group 1 moreover comprises one pair of collecting devices 10,10a, designed to collect the infusion product from their corresponding bottles 103,104 and to convey it to the aforesaid volumetric pumps 2,2a.

Each collecting device 10,10a respectively comprises a hollow needle 11,11 a, fixed to a body 12,12a fit to penetrate the bottle 103,104, in a known way, through a gummy closure provided on the bottles neck. The hollow needles 11,11a communicate with respective dropping devices 13,13a. These latter are in turn fixed to said body 12,12a and destined to receive a reserve of infusion product. Moreover, each dropping device 13,13a communicates with a corresponding connecting hoses 20,20a, by means of their own connecting joints 19,19a, in order to convey the infusion product to the cylinders 3,3a. For this purpose, suitable cylinder inlets 6,6a are provided in the block 50, which receive the above cited connecting joints 19,19a.

The collecting devices 10,10a also comprise compensating valves 14,14a, made in the body 12,12a and fit to allow air to enter the aforesaid bottles 103,104 in order to substitute the outflowing infusion product.

The collecting devices 10,10a are movably mounted on relative supports 118,118a provided in the front wall 108, near the bottles support 102, so as to allow the needles 11,11 a to be perfectly aligned with the bottle necks.

Between each cylinder inlet 6,6a and its respective cylinder 3,3a there is interposed a unidirectional input valve 7,7a, fit to allow the infusion product to flow only on an input direction in the cylinders 3,3a. The above input valves 7,7a are advantageously of a vertical chamber type, provided with a sealing sphere operating by gravity.

At the output side of the cylinders 3,3a there are also provided unidirectional output valves 9,9a, which are vertical chamber, sealing sphere valves as the above input valves 7,7a. They are fit to allow the infusion liquid to flow only toward an output direction from their relative cylinders 3,3a.

The unidirectional output valves 9,9a communicate with corresponding output joints 8,8a, which in turn merge to a single output pipe 30. This latter comprises a rigid portion 35 and an elastic portion 36, connected to the rigid one and provided with a standard joint 37 for connecting with standard needles for injecting the infusion liquid into a vein.

The connecting conduits 20,20a also communicate one each other by means of a further joint 25, provided in an intermediate position between the collecting devices 10,10a and the corresponding input joints 6,6a.

The pumping group 1 also comprises pressure detecting means 3 1, arranged at the rigid portion of the output pipe 30 and fit to detect the liquid pressure downstream of the volumetric pumps 2,2a, and upstream of the pressure drop induced by the subsequent output pipe and injecting needle.

Advantageously, the aforesaid pressure detecting means 31 comprise a cylinder-piston group, whose stem is arranged to act on a pressure sensor 32, which preferably consists of a load cell. Said cylinder-piston group is arranged in a suitable operating position on the front wall 108. The above cited piston stem is fit to act against the load cell 32 with an action which is proportional to the liquid pressure in the output pipe 30. The load cell 32 is suitably connected in a known way to a control unit of the apparatus 100, not shown, and its pressure values are handled by a control program residing in that control unit.

The infusion apparatus 100 moreover comprises actuating members 40, fit to reciprocally actuate the pistons 4,4a, according to pre-defined operating modes which are controlled by the above control unit. The actuating members 40 are arranged inside the casing 101, more precisely in its lower part, at an extension 110 of the same casing 101, so as to be placed in a substantially vertical alignment with the volumetric pumps 2,2a.

More particularly, the aforementioned actuating members 40 comprise a pair of cams 41,41a, eccentrically and vertically mounted on a horizontal shaft 42. Each cam 41,41a is provided with a respective closed-path cam track 43,43a obtained in the counter-facing sides thereof. One pair of cam following rollers 44,44a is engaged in the corresponding cam tracks 43,43a. These rollers 44,44a are mounted at one end of respective actuating rods 45,45a, which are arranged vertically, and respectively aligned with the aforesaid pistons 4,4a. At their opposite ends, the rods 45,45a are moreover removably connected to said pistons 4,4a by means of slide couplings 46,46a. The rods 45,45a come out of the casing 101 by means of suitable guiding bushings 111,111 a located in the same casing 101.

The actuating members 40 also comprise a motor-reduction gear assembly 48, mechanically connected to the shaft 42 and fit to rotationally drive it with rotation speeds and duration defined by the above mentioned control unit.

The cams 41,41a have a track path designed to operate their corresponding piston 4,4a in a suitably offset way, and preferably in a way such that one piston is in its bottom dead center the other one is in its top dead center.

The infusion apparatus 100 also comprises clamping means 120,120a, arranged on the front wall 108 in an intermediate position between the collecting devices and the volumetric pumps 2,2a, fit to engage the connecting pipes 20,20a, when driven by the control unit, for interrupting the infusion liquid flow in its corresponding pipe.

More particularly, the clamping means 120,120a comprises, for eache connecting pipe 20,20a, a fixed block 121,121a and a movable block 122,122a, among which there is interposed a portion of a corresponding connecting pipe 20,20a. Each movable block 122,122a is eccentrically mounted on a rotatable plate 124,124a which is axially mounted on a respective shaft 125,125a. These latter extend inside the casing 101, through the front wall 108. They are connected to driving members 126,126a of the same rotatable plate 124,124a. Said driving members 126,126a consist of electromagnetic actuators 127,127a and of corresponding return springs 128,128a, fit to rotatably actuate the plates 124,124a between opening positions A and closing position B of their respective connecting pipes.

On the front wall 108 the infusion apparatus 100 moreover comprises bubble detecting means 60, fit to detect air bubbles in the elastic portion of the output pipe 30. Said bubble detecting means 60 substantially comprises an ultrasonic type air bubbles detector, operating on a portion of the aforesaid output pipe interposed thereunder. This detector, whose structure and operating mode is perfectly known as it is commonly available for sale, is connected to the apparatus control unit, and it is adjusted so as to reveal even small air bubbles in the output pipe, in order to allow the same control unit to stop the infusion operations accordingly.

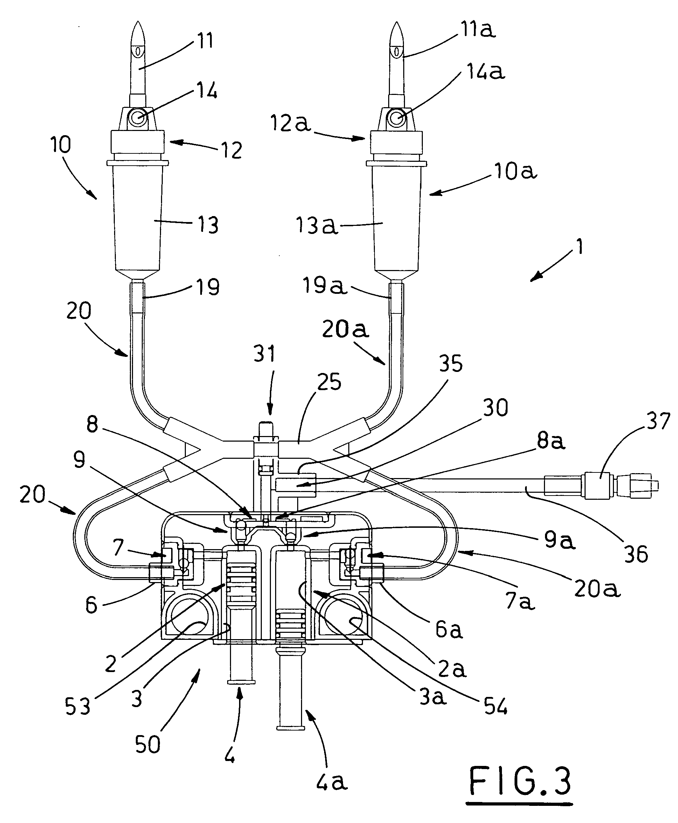

By way of an example, in the following an operating mode of the infusion apparatus 100 will be described, with reference to figures 1 to 3, and to a starting condition wherein bottles 103,104 full of infusion product, e.g. a contrast means for radiological investigations, are mounted on their support, and with the hollow needles 11,11 a of the collecting devices 10,10a being inserted into the aforesaid bottles so as to put in liquid communication the inside of said bottles with the dropping devices 13,13a. The clamps 120,120a are in their closed position B and pinch their respective connecting pipes 20,20a, so obstructing the same. The motor-reduction gear assembly 48 is not working, and the volumetric pumps 2,2a are not working as well.

In order to carry out an infusion session, one of the clamps, e.g. clamp 120, is led to its opening position A, and the motor-reduction gear assembly 48 is started. In this way the cams 41,41a begin to rotate and draw the cam follower rollers 44,44a. These latter drive the rods 45,45a and the pistons 4,4a by a reciprocally motion. Pistons 4,4a begin to exert a sucking-pressing action, respective on the connecting pipes 20,20a and on the output pipe 30.

Infusion product is then sucked from bottle 103. This product enters both the cylinders 3,3a through connecting pipes 20,20a and through the connecting joint 25, and then through the input joints 6,6a and the input valves 7,7a. Inside the cylinders 3,3a the infusion product is compressed and the sent to the output pipe 30, and then to the hollow needle to be injected in a vein.

The sucking and compressing phases offset on pistons 4,4a allows the output pipe to be advantageously fed with infusion product in a substantially continuous mode, the specifically designed track path of cams 41,41a allowing the pistons speed and acceleration to be adjusted so as to minimize the pressure pulses due to the substantially discontinuous operating mode of each piston 4,4a.

When the bottle 103 reaches its minimum level, under which continuing the infusion session would not possible, the collecting device 10 is then closed, driving the clamp 120 to its closing position B, and driving the other clamp 120a to its opening position A, in order to allow more infusion product to be collected from bottle 104. By this way, the empy bottle 103 can be replaced with a full one, with no interruption of the infusion operations.

It is therefore evident that, if required, the product collecting could be carried out from both bottles at the same time, simply leaving both clamps 120,120a in their opening position A.

Should any air bubble form in the infusion product flow, e.g. due to a possible malfunctioning in the liquid level sensors provided in the dropping devices 13,13a, said bubble is detected when it reaches the bubble detector 60. This event causes the pumping operation to be suddenly stopped. Said operations shall resume only after the problem has been fixed.

In any case, one of the most characterizing features of the present invention is that pumping groups which are disposable, or at least easily replaceable, are provided for the infusion apparatus 100. This allows the same apparatus to be always immediately available for a new infusion session.

## Claims

1. Apparatus for carrying out haematic infusions, **characterized in that** it comprises: a casing (101); a support (102) for at least one container (103) for infusion product, fixed to said casing (101); a pumping group (1), supported by said casing (101) and comprising: at least one reciprocating volumetric pump (2), provided with a cylinder (3) receiving a piston (4), which is reciprocally sliding in said cylinder (3) in a liquid tight mode; at least one collecting device (10) for collecting infusion product from said container (103), fit to penetrate said container (103) for collecting infusion product therefrom; at least one connecting pipe (20), extending from said collecting device (10) and an input joint (6) made in said volumetric pump (2), and fit to convey the infusion product from said collecting device (10) down to inside said cylinder (3); a unidirectional input valve (7), interposed between said input joint (6) and cylinder (3), fit to allow said infusion product to flow only toward said cylinder (3); at least one output pipe (30), connected to an output joint (8) of said volumetric pump (2) and fit to convey said infusion product from said cylinder (3) toward vein injection means; a unidirectional output valve (9), interposed between said cylinder (3) and said output joint (8), fit to allow said infusion product to flow only away from said cylinder (3); said infusion apparatus (100) moreover comprising actuating members (40), mechanically connected to said piston (4) and fit to reciprocally drive the same according to pre-defined operating modes.

2. Apparatus according to Claim 1, **characterized in that** said pumping group (1) comprises one pair of said volumetric pumps (2,2a), having their respective cylinders (3,3a) arranged side by side, their respective ouptut joints (8,8a) communicating one each other, and also communicating with said output pipe (30), each volumetric pump (2,2a) being provided with respective input valves (7,7a) and with respective output valves (9,9a), with respective input joints (6,6a) communicating with said collecting device (10,10a) by means of respective connecting pipes (20,20a) and with said actuating means (40) being designed to reciprocally drive the pump pistons (4,4a) inside their respective cylinders (3,3a).

3. Apparatus according to Claim 1 or Claim 2, **characterized in that** said pumping group (1) is removably fixed to a front wall (108) of said casing (101) and to said actuating members (40), and it is entirely replaceable with another pumping group (1) of the same type.

4. Apparatus according to Claim 1 or Claim 2, **characterized in that** said input valves (7,7a) comprise a vertical chamber and a sealing sphere actuated by gravity.

5. Apparatus according to Claim 1 or Claim 2, **characterized in that** said output valves (9,9a) comprise a vertical chamber and a sealing sphere actuated by gravity.

6. Apparatus according to Claim 1 or Claim 2, **characterized in that** said volumetric pumps (2,2a), said input valves (7,7a), said output valves (9,9a), said input joints (6,6a) said output joints (8,8a) and said ouptut pipe (30) are made in a single block (50) of plastic material.

7. Apparatus according to Claim 6, **characterized in that** said block (50) of plastic material comprises two moulded half-shells (51,52), rigidly fixed one each other.

8. Apparatus according to Claim 1 or Claim 2, **characterized in that** said pumping group (1) moreover comprises pressure detecting means (31) arranged at said output pipe (30) and fit to detect the pressure of the infusion product flowing inside said pipe (30).

9. Apparatus according to Claim 8, **characterized in that** said pressure detecting means (31) comprises a cylinder-piston group, whose stem is fit to act on a pressure sensor (32), which is mounted in a suitable position on the front wall (108), with an action which is substantially proportional to the pressure exerted by the liquid inside the output pipe (30).

10. Apparatus according to Claim 1 or Claim 2, **characterized in that** said collecting device (10,10a) comprise a hollow needle (11,11a), which is fixed to a body (12,12a) and fit to penetrate a corresponding cited container (103,104), said needle moreover communicating with a dropping device (13,13a) in turn fixed to said body (12,12a) and fit to receive a reserve of infusion product, said dropping device (13,13a) communicating with a respective connecting pipe (20,20a) by means of a connecting joint (19,19a).

11. Apparatus according to Claim 10, **characterized in that** said collecting devices (10,10a) moreover comprise a compensating valve (14,14a), made in the above cited body (12,12a) and fit to allow external air to reach said container (103,104) for compensating the internal container pressure.

12. Apparatus according to Claim 2, **characterized in that** it also comprises a joint (25), fit to put into communication said connecting pipes (20,20a), at an intermediate position between said collecting devices (10,10a) and the corresponding input joints (6,6a).

13. Apparatus according to Claim 1 or Claim 2, **characterized in that** it moreover comprises clamping means (120,120a), arranged on a front wall (108) of said casing (101), so as to engage said connecting pipes (20,20a) at an intermediate position between said collecting devices (10,10a) and said volumetric pumps (2,2a) for stopping the infusion product flow in at least one of the same connecting pipes, driven by a control unit.

14. Apparatus according to Claim 13, **characterized in that** said clamping means (120,120a) comprises a fixed block (121,121a) and a movable block (122,122a), among which is interposed a portion of said respective connecting pipes 820,20a), said movable block (122,122a) being eccentrically fixed on a corresponding, rotatable plate (124,124a) axially mounted on a shaft (125,125a) extending inwards of said casing (101), and driving members (126,126a) for said rotatable plate (124.124a), fit to operate the same between an opening position A and a closing position B of their respective cited connecting pipes (20,20a).

15. Apparatus according to Claim 14, **characterized in that** said driving members (126,126a) comprise an electomagnetic actuator (127,127a), arranged inside the casing (101), fit to rotatably operate said shaft (125,125a).

16. Apparatus according to Claim 1, **characterized in that** said actuating means (40) comprise: at least one cam (41), mounted on a shaft (42) and provided with a closed cam track (43) wherein a cam follower roller (44) is engaged; one actuating rod (45), mechanically connected at one end to said cam follower roller (44) and at the opposite end to said piston (4); a motor-reduction gear assembly, mechanically connected to said shaft (42) and fit to rotatably drive it with predefined speed and duration.

17. Apparatus according to Claim 2, **characterized in that** said actuating members (40) comprise: one pair of cams (41,41a), mounted on a shaft (42) and provided with respective closed cam tracks (43,43a), wherein corresponding cam follower rollers (44,44a) are engaged; actuating rods (45,45a), connected at one end to a respective cam follower roller (44,44a) and at the opposite end to a respective piston (4,4a); a motor-reduction gear assembly (48), mechanically connected to said shaft (42) and fit to rotatably drive the same with pre-defined speed and duration, said cams (42,42a) being made so as to allow said pistons (4,4a) to be operated in an offset mode.

18. Apparatus according to Claim 16 or Claim 17, **characterized in that** said actuating rods (45,45a) comprise, at their end connected to said pistons (4,4a), corresponding slide couplings (46,46a), fit to allow the same to be disconnected from their respective pistons (4,4a).

19. Apparatus according to Claim 1 or Claim 2, **characterized in that** it moreover comprises bubble detecting means (60), fit to detect air bubbles inside said output pipe (30), and to allow the infusion session to be stopped when said air bubbles are detected.

20. Apparatus according to Claim 1, **characterized in that** said detecting means (60) are ultrasonic detecting means.
